Europäisches Patentamt

European Patent Office

Office européen des brevets

(11) Veröffentlichungsnummer: **0 001 224**
**A1**

(12) **EUROPÄISCHE PATENTANMELDUNG**

(21) Anmeldenummer: **78100762.0**

(22) Anmeldetag: **28.08.78**

(51) Int. Cl.²: **G 01 N 33/16**
**C 08 F 8/00**

(30) Priorität: **19.09.77 GB 38893/77**

(43) Veröffentlichungstag der Anmeldung:
**04.04.79 Patentblatt 79/7**

(84) Benannte Vertragsstaaten:
**BE CH DE FR GB NL SE**

(71) Anmelder: **F.Hoffmann-La Roche & Co.**
**Aktiengesellschaft**
**Abt. VIII-Pt**
**CH-4002 Basel(CH)**

(72) Erfinder: **Fischer, Ernst Alfred, Dr.**
**Rüttihardstrasse 2**
**CH-4142 Münchenstein(CH)**

(74) Vertreter: **Henrich, Werner et al,**
**Grenzacherstrasse 124 Postfach 601**
**CH-4002 Basel(CH)**

(54) **Verfahren zur Herstellung eines wasserunlöslichen, immunologisch-diagnostischen Reagenzes.**

(57) Es wird beschrieben ein Verfahren zur Herstellung eines wasserunlöslichen, immunologisch-diagnostischen Reagens mit einem spezifischen Gewicht von etwa dem von Wasser, das diskrete Teilchen eines immunologisch inerten, carboxylierten Latex-Polymers aufweist, das über eine Amidbindung ein bekanntes, immunologisch aktives Material ankondensiert aufweist, wobei in einer ersten Stufe das immunologisch inerte, carboxylierte Latex-Polymer, von irgendwelchen verunreinigenden Aminoverbindungen praktisch frei, mit einem wasserlöslichen Aktivierungsmittel gemischt und in einer zweiten Stufe das bekannte immunologisch aktive Material zugesetzt wird.

EP 0 001 224 A1

Croydon Printing Company Ltd.

RAN 4093/34

F. Hoffmann-La Roche & Co. Aktiengesellschaft, Basel/Schweiz

Verfahren zur Herstellung eines wasserunlöslichen, immunologisch-diagnostischen Reagens.

Die Erfindung bezieht sich auf ein Verfahren zur Herstellung eines wasserunlöslichen, immunologisch-diagnostischen Reagens, insbesondere auf ein verbessertes Verfahren zum chemischen Binden immunologisch aktiver Materialien an ein immunologisch inertes, carboxyliertes Latex-Polymer.

Carboxylierte Latex-Polymere, insbesondere carboxylierte Styrol-Butadien-Latices haben sich als Träger für immunologisch aktive Materialien (Antigene oder Antikörper) bei Immunoassay-Systemen auf der Grundlage der Latex-Agglutination als sehr brauchbar erwiesen. Wie z.B. in der deutschen Patentanmeldung P 2203377 offenbart, wird beim herkömmlichen Verfahren zum kovalenten Kuppeln des immunologisch aktiven Materials dieses mit dem Latex gemischt und danach das Gemisch mit einem wasserlöslichen Carbodiimid behandelt.

Hen/14.8.1978

Der erste Schritt beim herkömmlichen Verfahren ist die Adsorption des immunologisch aktiven Materials an die Latex-Kügelchen. Beim zweiten Schritt wird ein chemisches Kupplungsmittel (Carbodiimid) zugesetzt, das zwischen dem Latex und dem adsorbierten, immunologisch aktiven Material kovalente Bindungen einführt.

Das bekannte Verfahren hat den Nachteil, daß die an der Oberfläche des immunologisch aktiven Materials freiliegenden reaktiven Gruppen auch aktiviert werden, und daraus resultieren intramolekulare und intermolekulare Querverbindungen bzw. Vernetzungsstellen. Daher ist die Konzentration des Carbodiimids sehr kritisch, ein Überschuß beeinträchtigt die immunologische Aktivität. Dies ist besonders von Nachteil, wenn Antikörper an die Latex-Kügelchen gekuppelt werden, da jede Veränderung ihrer aktiven Stellen ihr Bindungsvermögen für Antigene verschwinden läßt oder stark verringert.

Die Erfindung bezieht sich auf ein Verfahren zum Kuppeln immunologisch aktiver Materialien an carboxylierte Latices über aktivierteEster und umfaßt eine chemische Aktivierung nur des Latex-Trägers. Dadurch wird eine unkontrollierte Modifizierung des immunologisch aktiven Materials vermieden, und es werden Reagentien mit einem hohen Wert an biologischer Aktivität erhalten.

Im einzelnen bezieht sich die Erfindung auf ein Verfahren zur Herstellung eines wasserunlöslichen, immunologisch-diagnostischen Reagens mit einem spezifischen Gewicht von etwa dem des Wassers, das diskrete Teilchen eines immunologisch inerten, carboxylierten Latex-Polymers aufweist, das über eine Amidbindung ein bekanntes, immunologisch aktives Material ankondensiert aufweist, dadurch gekennzeichnet, daß in einer ersten Stufe das von irgendwelchen kontaminierenden Aminoverbindungen praktisch freie, immunologisch inerte, carboxylierte Latex-Polymer mit einem wasserlöslichen Aktivierungsmittel gemischt und in einer zweiten Stufe das bekannte, immunologisch aktive Material zugesetzt wird.

Typische geeignete carboxylierte Latex-Trägerteilchen sind solche, die in Form einer wässrigen Latex-Suspension im Handel sind, gewöhnlich in Konzentrationen von etwa 40 bis etwa 60 % an Feststoffen. Viele Typen carboxylierter Latex-Polymerer eignen sich zur erfindungsgemäßen Verwendung, vorausgesetzt, sie erfüllen die oben aufgeführten Kriterien. Die Erfindung umfaßt die Verwendung aller geeigneter carboxylierter Latices.

Im Rahmen der Erfindung umfaßt der Ausdruck "carboxylierte Latex-Polymere" solche Latex-Polymeren, die wasserunlöslich sind, eine Teilchengröße im Bereich von etwa 0,01 bis etwa 0,9 µm, ein spezifisches Gewicht nahe dem von Wasser aufweisen, so daß nach dem Kuppeln mit dem immunologisch aktiven Material das spezifische Gewicht der Teilchen etwa 1,0 (0,95 bis 1,05) beträgt, was es ihnen ermöglicht, ständig in wässriger Suspension zu bleiben, die Teilchen müssen im Hinblick auf immonologisch-diagnostische Tests inert sein, sie müssen auch eine ausreichende Oberflächenladungsdichte haben, so daß ihre Abstoßungskräfte nach dem Kuppeln an das immunologisch aktive Material ausreichen, eine Aggregation zu verhindern.

Typische geeignete Latex-Polymere sind carboxylierte Styrol-Butadiene, carboxylierte Polystyrole, Acrylsäure-Polymere, Methacrylsäure-Polymere, Acrylnitril-Polymere, Acrylnitril-Butadien-Styrole, Polyvinylacetat-Acrylate, Polyvinylpyridine, Vinylchlorid-Acrylate und dergleichen. Einige im Handel erhältliche Latices, die sich zur erfindungsgemäßen Verwendung eignen, sind Amsco Res 4150, Amsco Res 3011 (American Mineral Spirits Co.); Dow Latex 815, Dow Latex 816, Dow Latex 620, Dow Latex 859, Dow CL 241 (The Dow Chemical Co.); Hycar 1512, Hycar 1877X8, Hycar 2600x120 (Goodrich Chemical Co.); Gelva 900, Lytron 612, Lytron 624 (Monsanto); Rhoplex LC40 3216, Amberlite Ultrafine (Röhm und Haas).

Bevorzugte carboxylierte Latex-Polymere sind praktisch frei von verunreinigenden Aminoverbindungen, insbesondere von Ammoniumionen.

Der Ausdruck "immunologisch aktives Material" bezieht sich auf Komponenten physiologischer Flüssigkeiten, Zell- und Gewebeextrakte, wofür ein immunologisches Gegenreagens zur Verfügung steht oder erzeugt werden kann. Typische immunologische Materialien sind primäre Amine, Aminosäuren, Peptide, Proteine, Lipoproteine, Glykoproteine, Sterine, Steroide, Lipoide, Nukleinsäuren, Enzyme, Hormone, Vitamine, Polysaccharide und Alkaloide.

Beispiele für solche immunologisch aktiven Substanzen sind in der folgenden Tabelle angegeben:

## Tabelle I

I. <u>Mikroorganismen</u>

<u>Bakterien</u>

1. Gram-positive Kokken
   Streptokokken (pyogenes, fecalis und viridans)
   Staphylokokken (aureus und albus)
   Pneumokokken (D. pneumoniae)

2. Gram-negative Kokken
   Neisseria (gonorrhoeae und meningitidis)

3. Gram-positive aerobe Bazillen
   Bacillus anthracis
   Corynebacterium diphtheriae
   Erysipelothrix
   Listeria monocytogenes

4. Gram-positive anaerobe Bazillen
   Clostridia (botulinum, perfringens, welchii und
   tetani)

5. Gram-negative anaerobe Bazillen
   Bacteroides

6. Gram-negative Intestinum-Bazillen
   Escherichia
   Klebsiella
   Enterobacter
   Proteus
   Pseudomonas
   Salmonella
   Shigella

7. Gram-negative nicht-intestinale Bazillen
   Pasteurella (pestis und tularensis)
   Hemophilus influenzae
   Brucella (melitensis, abortus und suis)

Bordetella pertussis

Malleomyces

8. Spirochäten

Treponema pallidum

Leptospira

Borrelia

9. Mycoplasma

10. Mycobacteria

11. Vibrio

12. Actinomyces

Protozoen

1. Intestinale Protozoen

Amöben

2. Flagellaten

Trichomonas

Leishmania

Trypanosomen

Toxoplasma

3. Sporozoen

Plasmodien (vivax, falciparum, malariae und ovale)

4. Intestinale Nematoden

Maden- oder Springwürmer

Hakenwürmer

Peitschenwürmer

5. Gewebe-Nematoden

Trichinella

Filaria (Wuchereria bancroftii)

Dracunculus

6. Trematoden

Schistosomen

Eingeweide-Saugwürmer

Gewebe-Egel

7. Cestoden
   Bandwürmer

8. Toxoplasma (T. gondii)

<u>Fungi</u>

1. Sporotrichum
2. Cryptococcus
3. Blastomyces
4. Histoplasma
5. Coccidioides
6. Candida

<u>Viren und Rickettsien</u>

1. Rickettsien
2. Viren
   Hunde-Hepatitis
   Shope-Papillome
   Influenza A & B
   Hühnerpest
   Herpes simplex
   Adenoviren
   Polyome
   Rous' Sarkom
   Impfpocken
   Poliovirus
   Masern
   Hundestaupe
   Leukämie
   Mumps
   Newcastle-Krankheit
   Sendai
   ECHO
   Maul- und Klauenseuche
   Psittacosis
   Tollwut
   Extromelia
   Baumviren

II. Gewebe-Antigene, einschließlich organspezifische
    Antigene

    Polysaccharide
    Hyaluronidase
    Tetanus-Toxin
    Ei-Ovalbumin
    Ei-Serumalbumin

0001224

Niere
Leber
Haut
Herz (Myoglobin)
Magen-Darm-Trakt
Prostata
Embryo-Antigene (alpha 1 Fetoprotein)
Tumor-Antigene (Carcinoembryo-Antigen)
Muskel
Kollagen
Amyloid

III. Hormone

Hypophysen-Hormone
Insulin
Glucagon
Thyroid-Hormon
Choriongonadotropin
choriones Wachstumshormon - Prolactin
Human-Placenta-Lactogen

IV. Enzyme

Pankreas-Chymotrypsinogen
Procarboxypeptidase
Desoxyribonuclease
Ribonuclease
Glyceraldehyd-3-phosphat-dehydrogenase
Katalase
Peroxidase

V. Blutzellen-Antigene, Blutgruppensubstanzen und andere Isoantigene

Blutplättchen
Megakaryozyten
Leukozyten
Erythrozyten
Blutgruppensubstanzen
Forssmann-Antigen
Histokompatibilitäts-Antigene

VI. Plasma-Proteine

Fibrin und Fibrinogen
Plasminogen und Plasmin
Albumin
Immunoglobuline
$\alpha$-1-Antichymotrypsin
$\alpha$-1-Antitrypsin
Komplement-Faktoren
Ceruloplasmin
Gc-Globulin
Haptoglobin

α-2-Makroglobulin
ß-2-Mikroglobulin
Orosomucoid
Prealbumin
Transferrin

VII. Milch-Proteine

Lactoferrin
Lysozym
Sekret-IgA
Sekret-IgM
Sekret-Komponente

VIII. Speichel-Proteine

Sekret-IgA
Sekret-IgM
Sekret-Komponente

IX. Urin-Proteine

X. Pathologische Proteine

Myeloma-Protein
Makroglobulinaemische Proteine
Dysglobulinaemische Proteine
Bence Jones I, II-Proteine
C-reaktives Protein
Kryoglobuline

XI. Antikörper, einschließlich Autoantikörper

Antikernfaktor
Thyroid-Autoantikörper
Anti-Tamm-Horsfall-Protein
Kalt-Agglutinine
Rheumatoid-Faktor
Adrenal-Autoantikörper
Autoantikörper zu Magenwandzellen bei perniziöser
    Anämie
Anti-Colon
Anti-Leber
Anti-Niere
Autoantikörper zu Spermatozoen
Anti-Herz
Muskel-Autoantikörper bei Myasthenia gravis
Autoantikörper zu Nervengewebe
Autoantikörper gegen Fasergewebe und Gefäßkomponenten
Autoantikörper gegen Blutplättchen und Megakaryozyten
Antikörper gegen Trophoblasten
Antikörper zu Mikroorganismen
Antikörper zu tierischen Antigenen
Antikörper zu Arzneimitteln

ugte immunologisch aktive Materialien sind
ntar-lactogen), Albumin, Choriongonadotro-
; γ-Globulin, Antikörper gegen Albumin,

nologisch aktivem Material, das an die
erten Latex-Polymerträger gebunden ist,
etwa 0,01 bis 15 Gewichtsprozent. Jedes begisch aktive Material wird jedoch in einer
in der es bei einem diagnostischen Test
en eingesetzt wird, daher wird jedes Ma-
Iräger in einem für die speziellen Anfordeen Verhältnis kombiniert. Die Erfindung um-
Verwendung einer Menge an immunologisch akin Kombination mit einem immunologisch inierten Latex-Polymerträger, die ausreicht,
wirksames Reagens zu liefern.

Stufe des erfindungsgemäßen Verfahrens wird
es Latex-Polymer mit einem wasserlöslichen
l gemischt.

ang mit der Erfindung verwendete Aktivieeine Verbindung, die die Carboxylgruppen
-Gruppen zu überführen vermag, wobei R eine
de Gruppe ist, z.B. kann das Kupplungsmitsliches Carbodiimid, Woodwards Reagens K
1-isoxazolium-3'-sulfonat), ein wasserlösiat usw. sein.

ugte Aktivierungsmittel sind wasserlösliche
Formel
          R'-N=C=N-R"

Cycloalkyl mit 5 bis 6 Kohlenstoffatomen
it 2 bis 12 Kohlenstoffatomen, z.B. Äthyl, n-Propyl, Isopropyl, n-Butyl, sec.-Butyl, Isobutyl, tert.-
Butyl, Amyl, Hexyl, Heptyl, Octyl, Nonyl, Decyl, Undecyl
und Dodecyl, monoarylsubstituierte Niederalkylreste, z.B.
Benzyl-, $\alpha$- und ß-Phenyläthyl, Monoarylreste, z.B. Phenyl,
Morpholino, Piperidyl, Morpholinyl-substituierte Niederalkylreste, z.B. Äthylmorpholinyl, piperidylsubstituierte
Niederalkylreste, z.B. Äthylpiperidyl, Diniederalkylamino,
Niederalkylreste, Pyridyl-substituierte Niederalkylreste,
z.B. $\alpha$-, ß- und $\gamma$-Methyl- oder -Äthylpyridyl sind, deren
Säureadditionssalze und quaternären Amine.

Der Träger und das Aktivierungsmittel werden erfindungsgemäß in wässrigem Medium, vorzugsweise bei Raumtemperatur
(etwa 20 bis etwa 25$^{o}$C) umgesetzt. Temperaturen von etwa
5 bis etwa 40$^{o}$C sind jedoch für die Reaktion geeignet. Die
Menge an Aktivierungsmittel kann innerhalb breiter Grenzen
variieren und hängt u.a. von der Dichte der Carboxylgruppen
und von der Gegenwart zusätzlich negativ geladener Gruppen,
wie Sulfonatgruppen, an der Oberfläche des Latex ab. Im
allgemeinen sind etwa 0,005 bis etwa 6,0 Gewichtsprozent
eines wasserlöslichen Aktivierungsmittels, bezogen auf das
Gewicht der Teilchen, geeignet, gewöhnlich jedoch werden
etwa 0,05 bis 2,0 Gewichtsprozent verwendet.

Der pH der Reaktion ist von Bedeutung und sollte vorzugsweise zwischen 2 und 7 liegen, wobei ein pH zwischen 4 und
5 besonders bevorzugt wird.

Um den pH zwischen diesen Grenzwerten während der Umsetzung
zu halten, wird der pH der Latex-Suspension vorzugsweise
auf zwischen 4 und 5 voreingestellt.

In einer zweiten Stufe des erfindungsgemäßen Verfahrens
wird der aktivierte Latex mit einem immunologisch aktiven
Material umgesetzt. Die Reaktion erfolgt vorzugsweise bei

... ... ... (etwa 20 bis etwa 25°C). Temperaturen von
... ... etwa 40°C sind jedoch auch geeignet.

... ... Reaktion ist von Bedeutung, da er nicht so sein
... ... er irgendwelche Protein-Reaktionskomponenten
... ... Gewöhnlich ist ein pH von 5 bis 9,5 geeignet.
... ... sind durch die Verwendung geeigneter Puffersy-
... ... erhalten, wie durch Borat- oder Phosphatpuffer
... ... gleichen.

... ... lierten Latexpolymeren werden vorzugsweise zur
... ... irgendwelcher verunreinigender Aminoverbindungen,
... ... ammoniumionen, behandelt. Bei einer bevorzugten Aus-
... ... wird das carboxylierte Latex-Polymer zuerst
... ... mit Wasser gewaschen und dann mit einem Ionenaus-
... ... behandelt, das die Ammoniumionen gegen nicht
... ... ionen, wie Natrium oder Kalium, austauscht.

... ... das Produkt einmal gebildet, kann es bei spezifi-
... ... diagnosetests unter Anwendung immunologischer Prin-
... ... verwendet werden. Es kann in jeder geeigneten Kon-
... ... in Abhängigkeit vom spezifischen Test verwendet
... ... Konzentrationen von etwa 0,5 bis etwa 2,5 Gewichts-
... ... sind jedoch geeignet, wobei 1,0 Gewichtsprozent be-
... ... wird.

... ... dem erfindungsgemäßen Verfahren erhaltenen Reagen-
... ... zur Bestimmung immunologisch aktiver Materialien
... ... direkten oder indirekten (Hemmung) Agglutinations-
... ... bei der in der deutschen Patentanmeldung P 2741956
... ... kinetischen photometrischen Methode verwendet

... ... direkten Test werden die Probe und die Latexteilchen,
... ... Gegenreagens für das immunologisch aktive Mate-

rial, das bestimmt werden soll, überzogen sind, gemischt
und die Agglutination visuell oder photometrisch beobachtet. Im Falle einer Agglutination ist der Test positiv.

Beim indirekten Test wird die Probe mit dem Gegenreagens
(z.B. Antiserum) für das zu bestimmende Material und dann
mit Latexteilchen, die mit dem gleichen Material überzogen
sind, gemischt. Die Agglutination wird visuell oder photometrisch beobachtet. Tritt keine Agglutination ein, ist
der Test positiv.

Die nach dem erfindungsgemäßen Verfahren erhaltenen Reagenzien können für Handelszwecke bequem verpackt werden, z.B.
in einem Diagnosereagenziensatz.

Die folgenden Beispiele veranschaulichen die Erfindung.

## Beispiel 1

Vorbehandlung von Latex Dow CL 241 und Kuppeln von Rinderserumalbumin (BSA)

125 ml einer handelsüblichen Suspension von Dow CL 241
(carboxyliertes Styrol-Butadien-Copolymerisat, etwa 50 %
Feststoffe) wurden mit 375 ml Wasser gemischt und 0,5 h
auf einem Magnetrührer gerührt. Dann wurde 60 min bei etwa
50 000 g zentrifugiert. Der Rückstand wurde in 500 ml $H_2O$
erneut suspendiert und noch dreimal zentrifugiert. Dann
wurde er in Wasser aufgenommen und der Feststoffgehalt auf
8 % eingestellt. 100 ml der 8 %igen Latex-Suspension wurden fünfmal mit 10 g nassem Dowex AG 50-X8, 200-400 mesh,
Bio Rad (Styrol-Divinylbenzol-Polymerlatex mit Sulfonsäuregruppen am Kern) behandelt, das rückgeführt wird und in
die Natriumform überführt worden war. Jedesmal wurde der
pH der Suspension auf 4,75 eingestellt.

Unter raschem Rühren wurde eine Lösung von 10 mg 1-Cyclohexyl-3-/2-morpholinyl-(4)-äthyl/-carbodiimid-metho-p-
toluolsulfonat (CMC) in 2 ml Wasser zu 6 ml ionenausgetauschtem, auf pH 4,75 eingestelltem Latex (78 mg/ml) gegeben. Nach 3 min wurden 2 ml einer 1 %igen Rinderserumalbuminlösung zugesetzt. Nach der Zugabe des Proteins betrug der pH der Suspension 5,7. Das Gemisch wurde bei Raumtemperatur etwa 12 h gerührt. In dieser Zeit verschob sich
der pH auf 6,75. Der Latex wurde dann 40 min bei 44 000 g
zentrifugiert. Die überstehende Flüssigkeit wurde verworfen,
das Pellet in Wasser (insgesamt etwa 40 ml) suspendiert, mit
einem Mischer homogenisiert und erneut zentrifugiert. Das
Waschen wurde viermal mit 0,1 m Boratpuffer vom pH 8,5 wiederholt. Nach zweimaligem Waschen mit destilliertem Wasser
wurde der Latex in 20 ml destilliertem Wasser aufgenommen.
Er wurde durch Zusatz von 40 µl 20 %iger Natriumazidlösung
stabilisiert.

Objektträger-Test

20 µl Ratten-Anti-BSA-Serum, in phosphatgepufferter Salzlösung (PBS) 1:400 verdünnt, werden mit 60 ml des gleichen
Puffers und 20 µl der obigen BSA-Latexsuspension gemischt:
Nach etwa 35 s wird eine Agglutination sichtbar.

Diese Agglutination kann durch Mischen von 20 µl Antiserum
(1:400 in PBS) zuerst mit 30 µl PBS und 30 µl des gleichen
Puffers mit 3 µg BSA spezifisch gehemmt werden. Für mehr als
4 min ist kein Anzeichen einer Agglutination festzustellen.

Beispiel 2

Kuppeln von Schaf-Antihuman-IgG-Antikörpern an Dow CL 241

2,5 ml einer Lösung von 5,8 mg/ml Schaf-Antihuman-IgG-Antikörpern in 1:1 mit Wasser verdünnter phosphatgepufferter
Salzlösung wurden 2 h gegen 1 l 0,015 m Boratpuffer vom pH 8,5 dialysiert. Die geringfügige Fällung wurde abfiltriert, und aktivierter Latex, erhalten nach Beispiel 1, mit 2,5 mg CMC anstelle von 10 mg CMC, wurde dem Filtrat zugesetzt. Die Suspension wurde bei 4°C über Nacht gerührt, zentrifugiert und dreimal mit 0,015 m Boratpuffer gewaschen. Schließlich wurde sie in 20 ml Boratpuffer aufgenommen und durch Zugabe von 40 µl einer 20 %igen Natriumazidlösung stabilisiert.

## Objektträger-Test

20 µl der obigen Schaf-Antihuman-IgG-Latexsuspension werden mit 60 µl veronalgepufferter Salzlösung (3,13 mMol Barbital, 1,82 mMol Natriumbarbital, 0,15 Mol Natriumchlorid, pH 7,5) mit 0,3 µg Human-IgG gemischt. Spezifische Agglutination tritt innerhalb 30 s ein.

## Beispiel 3

### Kuppeln von Human-placentar-lactogen (HPL) an Dow CL 241

3 ml ionenausgetauschter Latex Dow CL 241 (77,6 mg/ml) wurden auf pH 4,75 eingestellt. Unter raschem Rühren wurde eine Lösung von 5 mg CMC in 1 ml Wasser der Suspension zugesetzt. Nach 3 min wurde 1 ml einer 1 %igen Human-placentar-lactogen-Lösung zugesetzt. Das Gemisch wurde bei Raumtemperatur über Nacht gerührt und dann 40 min mit 45 000 g zentrifugiert. Der Latex wurde zweimal mit 0,015 m Boratpuffer, pH 8,5, gewaschen, dann zweimal mit 0,1 m Boratpuffer gleichen pH-Werts. Nach zweimaligem Waschen mit destilliertem Wasser wurde der Latex in 10 ml destilliertem Wasser aufgenommen und durch Zusatz von 20 µl 20 %iger Natriumazidlösung stabilisiert.

## Objektträger-Test

20 µl Kaninchen-Anti-HPL-Serum, mit PBS 1:20 verdünnt, wur-

den mit 60 µl des gleichen Puffers und 20 µl der obigen HPL-Latexsuspension gemischt. Agglutination trat innerhalb 5 bis 10 s ein. Diese Agglutination kann durch Mischen von 20 µl Antiserum (1:20 in PBS) zuerst mit 30 µl PBS und 30 µl des gleichen Puffers mit 3 µg HPL spezifisch gehemmt werden. Für mehr als 1 min ist überhaupt keine Agglutination zu beobachten.

Patentansprüche

1. Verfahren zur Herstellung eines wasserunlöslichen,
   immunologisch-diagnostischen Reagens mit einem spezifischen Gewicht von etwa dem von Wasser, das diskrete
   Teilchen eines immunologisch inerten, carboxylierten
   Latex-Polymers aufweist, das über eine Amidbindung ein
   bekanntes, immunologisch aktives Material ankondensiert
   aufweist, dadurch gekennzeichnet, daß in einer ersten
   Stufe das immunologisch inerte, carboxylierte Latex-Polymer, von irgendwelchen verunreinigenden Aminoverbindungen praktisch frei, mit einem wasserlöslichen Aktivierungsmittel gemischt und in einer zweiten Stufe das
   bekannte immunologisch aktive Material zugesetzt wird.

2. Verfahren nach Anspruch 1, dadurch gekennzeichnet, daß
   das wasserlösliche Aktivierungsmittel in einer Menge
   von etwa 0,005 bis etwa 6 Gewichtsprozent verwendet
   wird.

3. Verfahren nach Anspruch 1 oder 2, dadurch gekennzeichnet, daß als wasserlösliches Aktivierungsmittel ein wasserlösliches Carbodiimid verwendet wird.

4. Verfahren nach einem der vorhergehenden Ansprüche, dadurch gekennzeichnet, daß als wasserlösliches Carbodiimid 1-Cyclohexyl-3-/2-morpholinyl-(4)-äthyl7-carbodiimid-metho-p-toluolsulfonat verwendet wird.

5. Verfahren nach einem der Ansprüche 1 bis 4, dadurch gekennzeichnet, daß das immunologisch inerte, carboxylierte Latex-Polymer mit einem spezifischen Gewicht von etwa dem des Wassers und einer Teilchengröße von etwa 0,01 bis etwa 0,9 µm verwendet wird.

6. Verfahren nach einem der vorhergehenden Ansprüche, dadurch gekennzeichnet, daß das immunologisch aktive Material in einer Menge von etwa 0,01 bis etwa 15,0 Gewichtsprozent an das immunologisch inerte Latex-Polymer gebunden verwendet wird.

7. Verfahren nach einem der vorhergehenden Ansprüche, dadurch gekennzeichnet, daß als immunologisch inertes carboxyliertes Latex-Polymer ein wasserunlösliches carboxyliertes Styrol-Butadien-Copolymerisat verwendet wird.

8. Verfahren nach einem der vorhergehenden Ansprüche, dadurch gekennzeichnet, daß als immunologisch aktives Material Human-Choriongonadotropin verwendet wird.

9. Verfahren nach einem der Ansprüche 1 bis 7, dadurch gekennzeichnet, daß als immunologisch aktives Material Humanalbumin verwendet wird.

10. Verfahren nach einem der Ansprüche 1 bis 7, dadurch gekennzeichnet, daß als immunologisch aktives Material denaturiertes $\gamma$-Globulin verwendet wird.

11. Verfahren nach einem der Ansprüche 1 bis 7, dadurch gekennzeichnet, daß als immunologisch aktives Material Human-placentar-lactogen verwendet wird.

12. Verfahren nach einem der Ansprüche 1 bis 7, dadurch gekennzeichnet, daß als immunologisch aktives Material ein Antikörper gegen Albumin verwendet wird.

13. Verfahren nach einem der Ansprüche 1 bis 7, dadurch gekennzeichnet, daß als immunologisch aktives Material ein Antikörper gegen IgA verwendet wird.

14. Verfahren nach einem der Ansprüche 1 bis 7, dadurch gekennzeichnet, daß als immunologisch aktives Material ein Antikörper gegen IgG verwendet wird.

15. Verfahren nach einem der Ansprüche 1 bis 7, dadurch gekennzeichnet, daß als immunologisch aktives Material ein Antikörper gegen IgM verwendet wird.

16. Verfahren nach einem der Ansprüche 1 bis 15, dadurch gekennzeichnet, daß das immunologisch inerte carboxylierte Polymer zum Entfernen irgendwelcher verunreinigender Aminoverbindungen vorbehandelt worden ist.

17. Verfahren nach Anspruch 16, dadurch gekennzeichnet, daß ein durch Waschen mit Wasser und Behandeln mit einem Ionenaustauscherharz vorbehandeltes immunologisch inertes carboxyliertes Polymer verwendet wird.

*  *  *

Europäisches Patentamt

**EUROPÄISCHER RECHERCHENBERICHT**

0001224

Nummer der Anmeldung

EP 78 10 0702

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US – A – 4 045 384 (L.C. DORMAN)<br><br>* Spalte 7-8, Beispiel 5; Ansprüche 1-3; Spalte 1, Zeilen 45-51 * | 1,3,4 |
| A | FR – A – 2 125 001 (HOFFMANN-LA ROCHE)<br><br>* Ansprüche 1-14 * | 1-10 |
| | DE – A – 2 812 845 (ORTHO DIAGNOSTICS)<br><br>* Beispeile 3,4,5; Seite 8, Zeilen 12-21; Ansprüche 11,14 * | 1,3,4 7,8 |
| E | & NL – A – 78 03209 | |
| E | & FR – A – 2 384 789 | |

**KLASSIFIKATION DER ANMELDUNG (Int.Cl.²)**

G 01 N 33/16
C 08 F 8/00

**RECHERCHIERTE SACHGEBIETE (Int.Cl.²)**

G 01 N 33/16

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 19-12-1978 | MEYLAERTS |

EPA form 1503.1  06.78